(19) Europäisches Patentamt
European Patent Office
Office européen des brevets

(11) Publication number: 0 021 505
A1

(12) # EUROPEAN PATENT APPLICATION

(21) Application number: 80200543.9

(51) Int. Cl.[3]: A 61 K 7/06

(22) Date of filing: 12.06.80

(30) Priority: 20.06.79 US 50375

(43) Date of publication of application:
07.01.81 Bulletin 81/1

(84) Designated Contracting States:
BE DE FR GB IT NL

(71) Applicant: THE PROCTER & GAMBLE COMPANY
301 East Sixth Street
Cincinnati Ohio 45202(US)

(72) Inventor: Donovan, James Harold
2489 Owlcrest Drive
Cincinnati Ohio 45231(US)

(74) Representative: Brooks, Maxim Courtney et al,
Procter & Gamble (NTC) Limited Whitley Road
Longbenton
Newcastle-upon-Tyne NE12 9TS(GB)

(54) **Hair conditioning product and treatment of hair therewith.**

(57) Hair conditioning agents, such as ditallow dimethylammonium chloride, are releasably affixed to one surface of a substrate. The substrate (1), which must be flexible, impervious, and nonabsorbent, is provided with embossments (3) and with a backing sheet. In use, the product is passed over and through the hair so that the hair conditioning agent is transferred from the product to the hair. Preferably, the hair has been shampooed and is still damp at the time the product is used. After it has been treated with the product, the hair is easier to comb and detangle when wet and exhibits, when dry, enhanced softness, luster, and manageability.

Fig. 1

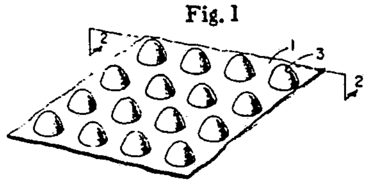


EP 0 021 505 A1

# HAIR CONDITIONING PRODUCT AND TREATMENT OF HAIR THEREWITH

The present invention relates to products and methods for conditioning hair. Specifically, the products comprise flexible, impervious, nonabsorbent substrates having chemical hair conditioning agents releasably affixed to one surface thereof. In the methods, the products are used to condition hair by contacting hair therewith, preferably after the hair has been shampooed, but before it has been dried.

It is a well known inconvenience that human hair becomes soiled during the course of day-to-day living. The soiling results from several different factors and the cooperation of these factors. For example, the build-up of oily, naturally-occurring material, commonly called sebum, on the hair causes it to have an unpleasant, dirty feel and an unattractive appearance. Sebum also contributes to enhanced soiling of the hair by attracting and holding materials from the surrounding atmosphere. Because of the dirty feel and unattractive appearance caused by the sebum and the atmospheric contaminants, many people consider regular, frequent shampooing to be a necessity.

Shampooing the hair does indeed remove contaminants and excess sebum, does reduce the dirty feel, and does enhance the appearance of the hair. However, the shampooing process in itself has disadvantages in that the hair is left in a wet, tangled, and generally unmanageable state. After drying, the hair is frequently

unmanageable and exhibits static fly-away. To improve the post-shampooing condition of the hair, a variety of approaches have been suggested. Many of these approaches involve the use of chemical hair conditioning agents or aids such as post-shampoo hair conditioning products, i.e. hair rinses. Because hair rinses typically function through the deposition of a film of material on the hair, the improvements they produce are frequently accompanied by new problems. Such new problems include the greasy feel of hair which has been washed and treated with a hair rinse and the sometimes enhanced contamination by atmospheric materials which results because of the use of the hair rinse. Also, hair rinses generally are liquid in nature and must be applied in a separate step following the shampooing of the hair. These rinses frequently must be left on the hair for some prescribed length of time and then the hair then must be rinsed to remove excess conditioning agent. This additional operation is, of course, time consuming and inconvenient.

Benjamin et al in U.S. Patent 4,149,551, issued April 17, 1979, describe a product and claim a process for the conditioning of just-shampooed hair. In general, they teach the conditioning of hair by rubbing it with an article comprised of a flexible substrate having releasably associated therewith a normally solid, substantially water insoluble hair conditioning agent. The benefits provided include reduced combing effort and detangling as well as enhanced postdrying softness, luster, manageability and reduced static fly-away. The flexible substrate Benjamin et al use is an absorbent paper or woven or nonwoven cloth preferably having an open or porous structure as opposed to a dense, kraft, paperlike structure.

Even though the products and methods described by Benjamin et al represent distinct improvements over previous hair conditioning products and techniques, not all problems associated with the conditioning of recently shampooed hair have been completely solved.

Accordingly, it is an object of the present invention to provide a product for conditioning hair by imparting to the hair combing, detangling, static flyaway, softness, luster, and manageability benefits.

It is another object of this invention to provide a hair conditioning product which is more efficient, effective, and convenient to use than previously available products.

A still further object of this invention is to provide a method for conditioning hair which has been recently shampooed.

These and other objects will become readily apparent from a reading of the description of invention which follows.

## Description of Invention

### Summary of the Invention

This invention provides a product for conditioning hair, particularly recently shampooed hair, which product comprises a flexible, impervious, nonabsorbent, planar substrate having releasably associated with the first face thereof a hair conditioning agent and having affixed to the second face thereof a backing sheet. A method of conditioning hair, such as hair which has been recently shampooed but not yet dried, is also provided.

### Detailed Description of the Invention

While the specification concludes with claims particularly pointing out and distinctly claiming the

subject matter which is regarded as the invention, it is believed that the invention can be more readily understood by careful study of this specification and of the appended examples and drawings.

The product of this invention comprises three basic elements: a flexible, impervious, nonabsorbent substrate (hereinafter sometimes referred to simply as "substrate"); a hair conditioning agent (hereinafter sometimes referred to as "treatment agent", "treating agent", or simply "agent") applied to the first surface or face of the substrate; and a backing sheet adjacent and affixed to the second surface or face of the substrate. The necessary and desirable characteristics of each element, and the manner in which they cooperate one with another, are discussed in the following sections. Merely because this invention is described in terms of three basic elements, it should not be assumed that other auxiliary or ancillary constituents cannot or should not be present. Such auxiliary or ancillary constituents can be present so long as no violence is done to the fundamental nature of the invention.

The Substrate

The first of the three basic elements of this invention is a flexible, impervious, nonabsorbent substrate. This is the element to which the treating agent is initially applied and from which it is transferred to the hair of the subject. (As used herein, unless a specific definition to the contrary is given, the term "subject" refers to a person who uses the products and methods of this invention to condition her hair. Unless otherwise indicated, the subject will first dampen her hair, as by shampooing, and will then transfer treatment agent from a product of this invention

to her damp hair as by rubbing her hair with such product. Also as used herein, the feminine pronoun is to be interpreted as including the masculine gender within its ambit.)

The substrate can be any sheet, film, or other planar material that is flexible, impervious, and nonabsorbent. The material of the construction of the substrate is immaterial so long as it meets the enumerated requirements. Further, it can be one or several plies, preferably a single ply.

"Flexibility" is used herein to describe the quality or ability of a planar material to be deformed by moderate-to-light manual pressure. A flexible material also possesses the quality or ability of being deformed by moderate-to-light manual pressure into more or less conforming register with relatively small objects such as a lock of human hair.

"Impervious" is used herein to describe the quality or ability of a planar material to resist penetration by a fluid such as a liquid treatment agent or water. While it is recognized that in a strict, technical sense most planar materials have some finite permeability to one or more fluids, it is sufficient for the purposes of the present invention that the substrate be impervious in a practical sense, i.e., that it resist penetration by fluids during reasonable storage conditions and reasonable conditions of use.

"Nonabsorbent" is used herein to describe the quality or ability of a planar material to resist the incorporation of applied fluids into its matrix. In particular, "nonabsorbent" refers to the quality or ability of the substrate to resist absorption of water with which the hair of a subject is dampened and to resist the absorption of any applied treatment agent.

Typically, the substrate is a plastic film material such as polypropylene or, preferably, polyethylene. Preferred substrates are low density polyethylene films having a thickness of from about 0.014 to about 0.05 millimeter and a basis weight of from about 14.0 to about 60.0 grams per square meter. The plastic films used for the substrates can be made by any of the various techniques well known to those skilled in the art of plastic film manufacture. Depending upon the particular aesthetic properties which the maker of the product of this invention wishes to incorporate into the product, the substrate can be transparent or translucent and can optionally incorporate dyes and pigments.

In one embodiment, the substrate is a smooth plastic film. That is to say, each surface of the substrate is itself essentially planar and has an effective surface area of approximately one square meter per square meter of substrate. In other embodiments, some of which are preferred, the surface of the substrate can be modified so that the surface is no longer essentially planar. That is, at least one surface of the substrate will have an effective surface area of more than one square meter per square meter of substrate.

In a preferred embodiment, the plastic film comprising the substrate is embossed in any of numerous possible configurations. One purpose of the embossing is to increase the effective surface area of the substrate.

A preferred embossment geometry is shown in Figures 1 and 2. Figure 1 is a perspective view of a portion of a preferred substrate of this invention. Reference numeral 1 indicates generally the substrate. Reference numeral 3 indicates an embossment (or permanent deformation) in substrate 1. Figure 2 is a sectional view of

the embossed substrate of Figure 1 taken along the line 2-2 of Figure 1. The conical geometry of the embossment 3 is clearly shown in Figure 2. It should be noted that the apex 6 of the conical embossment 3 is shown as being hemispherical rather than sharply pointed as in a true cone. While sharply pointed apices are within the scope of this invention, the hemispherical apices shown more nearly represent the situation generally prevailing in actual practice. The embossed cones 3 have a base diameter indicated by d and a height indicated by h. The cones are separated by a distance s between their bases. The particular dimensions h, d, and s can be of any convenient value. Preferably, the diameter of the base is from about 0.3 to about 1.5 millimeters and the height is from about 0.10 to about 0.5 millimeter.

While the conical embossments can be distributed across the surface in a random manner, it is preferred that they be arranged in a regular pattern or a regular repeating pattern. Figures 5 and 6 are fragmentary plan views of substrate 1 which show the distribution of embossments generally. While for immediate purposes and discussion, reference numerical 3 can be considered to represent conical embossments, it can also represent the other embossment geometries discussed hereinafter. In Figure 5, the embossed cones 3 are arranged in regular ranks and files. The distance between bases of embossments 3 within a rank is shown by s and the distance between bases of embossments 3 within a file is shown by g. Dimensions s and g can be the same or they can be different; they can be of any convenient value.

In preferred forms of this particular embodiment, the distances (s and g) between the bases of the cones are from about 0 millimeter (i.e. the bases of the cones actually touch) to about 0.5 millimeter.

In Figure 6, the embossed cones 3 have been arranged so that cones in adjacent ranks are placed in offset files. The distances s and g have the same definitions as in Figure 5.

In especially preferred embodiments of the two arrangements shown in Figures 5 and 6, the spacings (s and g) between the embossed cones correspond approximately to the diameter of a small bundle comprising from about 5 to about 20 human hairs and are from about 0.1 to about 0.4 millimeter.

In another preferred embodiment, the substrate contains cylindrical embossments. Figure 3 is a sectional view of a substrate containing cylindrical embossments and is analagous to that shown in Figure 2 for conical embossments. The cylindrical embossments are indicated generally by reference numeral 4. The height of the cylinders is indicated by $h_1$, the base diameter by $d_1$, the apex diameter by $d_2$, and the spacing between bases of the embossed cylinders by $s_1$. In a preferred form of this embodiment, the cylinders are right cylinders; that is, $d_1$ and $d_2$ are essentially equal and are from about 0.10 to about 0.50 millimeter. In a related embodiment, the base and apex diameters $d_1$ and $d_2$ can be different. In this particular situation, $d_1$ is usually greater than $d_2$. When $d_1$ is greater than $d_2$, the embossment is technically not a cylinder, but is, rather, a frustrum of a cone. This particular geometry is described in this specification along with cylinders because, when the difference between $d_1$ and $d_2$ is small, on the order of 20% or less, the embossments usually visually resemble cylinders rather than frustrums of cones. As used in the specification, cylindrical embossments include frustrums of cones as last described.

Figure 4 is analogous to Figure 3 and is a sectional view of a substrate containing cylindrical embossments.

Here, the apex 5 of the embossment is generally an oblate spheriod having a radius of from about 25% to about 40% of $d_1$ rather than a flat plane as shown in Figure 3. The indicated dimensions have the same definitions as in Figure 3. Figure 4 represents an especially preferred embodiment of the substrate.

Figure 7 is also analogous to Figure 3 and represents another preferred embossment. Here, the total embossment 7 is an oblate spheriod having a height t and a base diameter d. Preferably, t is from about 0.07 to about 0.40 millimeter and d is from about 0.50 to about 1.30 millimeters.

Figures 5 and 6, as noted, represent the distribution of embossments generally on substrate 1. In the discussion above, reference numeral 3 indicated conical embossments; it can also represent the other embossment geometries discussed above.

As will be discussed in greater detail hereinafter, preferred embodiments of the present invention comprise substrates containing embossments because the embossments increase the surface area of the substrate available for coating with treatment agent, enhance the rate of transfer of treatment agent to a subject's hair, and separate the subject's hair into small bundles of hair, thereby increasing the uniformity of transfer of treatment agent to a subject's hair. They also enhance the visual and tactile aesthetic characteristics of the substrate.

One embossment geometry that can be used in the present invention is described in U.S. Patent 3,929,135 which was issued to Thompson on December 30, 1975 and which is incorporated herein by reference. One element of Thompson's absorptive structure is a fluid impervious topsheet material provided with tapered capillaries or orifices of critical opening dimensions and of critical

angles of taper. The topsheet described by Thompson can be used as the substrate of the present invention. While Thompson's topsheet actually contains holes or perforations within the embossed conical structure, the geometry of the embossments is such that liquids contacting the apex of an embossment will not enter the opening of the embossment or be transported through the plane of the topsheet. For practical purposes, the topsheet described by Thompson is impervious as the term is used in this specification.

Several embossment geometries have been described. It will be readily apparent to those skilled in the art that other geometries, designs, patterns and arrangements can be used without departing from the scope and spirit of this invention.

Embossments can be made into plastic films having planar surfaces by any of the various embossing and debossing techniques well known to those skilled in the art of plastic films.

Optionally, the surface of the plastic film can be roughened, as by providing it with nubbles or other minute surface features, to decrease its shine and to improve its visual aesthetic characteristics.

One additional requirement of the substrate is that it must be nonreactive with the treatment agent. In this context, "nonreactive" means that the substrate and the treatment agent do not interact in a manner producing deleterious results in either the substrate or the treatment agent. It is conceivable, and within the scope of this invention, that a beneficial interaction between the substrate and the treatment agent can and will occur in certain circumstances.

In length and width, the substrate can be any convenient size. It can be in the form of individual sheets or in the form of a continuous roll of material. In a preferred embodiment, an embossed substrate is provided in the form of a continuous length of material in roll form and is processed as hereinafter described.

<u>The Hair Conditioning Agent</u>

The second of the three basic elements of this invention is the hair conditioning agent, sometimes referred to as the treatment agent. Treatment agents are chemical compounds which are usually solid at temperatures below about 30°C, which are usually water insoluble, and which, when applied to a subject's hair, impart beneficial characteristics to the hair.

Included among the beneficial characteristics which the treatment agents provide are enhanced ease of combing of the hair not only when wet but also when dry; enhanced detangling of the wet hair; enhanced softness, body, fullness, luster, clean feel, manageability, and decreased static fly of a subject's hair after drying.

Suitable treatment agents are selected from the group consisting of quaternary ammonium salts, quaternary imidazolinium salts, sorbitan esters, fatty alcohols, polyhydric alcohol esters, fatty acid salts, aliphatic ethers, and mixtures thereof. The quaternary ammonium salts are preferred. In addition to their combing, detangling, softness, luster and manageability benefits, they provide excellent static flyaway benefits. The individual classes of hair conditioning agents are discussed in the following paragraphs.

Quaternary ammonium salts have the formula:

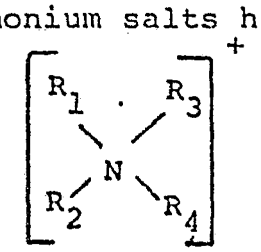 $X^-$

wherein $R_1$ is hydrogen, an aliphatic group of from 1 to 22 carbon atoms, or an aromatic, aryl or alkaryl group having 6 to 20 carbon atoms; $R_2$ is an aliphatic group having from 12 to 22 carbon atoms; $R_3$ and $R_4$ are each alkyl groups having from 1 to 3 carbon atoms; and X is an anion selected from halogen, acetate, phosphate, nitrate and methyl sulfate radicals.

Preferred quaternary ammonium salts are the dialkyl dimethyl ammonium chlorides, hwerein the alkyl groups have from 12 to 22 carbon atoms and are derived from longchain fatty acids, such as hydrogenated tallow. The term "tallow" refers to fatty alkyl groups derived from tallow fatty acids. Such fatty acids give rise to quaternary compounds wherein $R_1$ and $R_2$ have predominately from 16 to 18 carbon atoms. The term "coconut"refers to fatty acid groups derived from coconut oil fatty acids. Such fatty acids give rise to quaternary compounds wherein $R_1$ and $R_2$ have from 8 to about 18 carbon atoms, predominately from 12 to 14 carbon atoms.

Representative examples of quaternary ammonium salts useful in this invention include ditallow dimethyl ammonium chloride, ditallow dimethyl ammonium methyl sulfate, dihexadecyl dimethyl ammonium chloride, di(hydrogenated tallow) dimethyl ammonium chloride; dioctadecyl dimethyl ammonium chloride, dieicosyl dimethyl ammonium chloride; didocosyl dimethyl ammonium chloride, di(hydrogenated tallow) dimethyl ammonium acetate, dihexadecyl diethyl ammonium chloride, dihexadecyl dimethyl ammonium acetate, ditallow dipropyl ammonium phospahte, ditallow dimethyl ammonium nitrate, di(coconut-alkyl)dimethyl ammonium chloride; and stearyl dimethyl benzyl ammonium chloride.

Other quaternary ammonium salts useful herein are the compounds of the formula

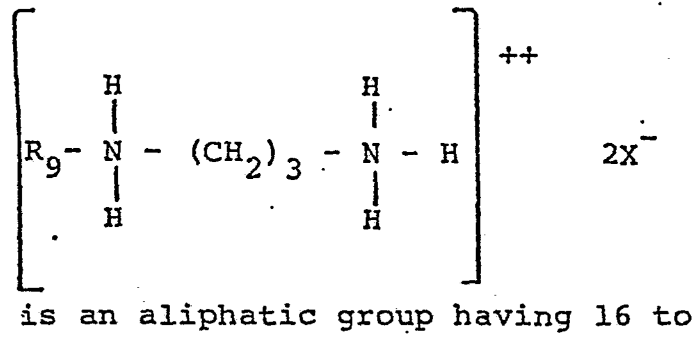

wherein $R_9$ is an aliphatic group having 16 to 22 carbon atoms and X is an anion as above defined. Tallow propanediamine hydrochloride is an example of this quaternary ammonium salt.

Quaternary imidazolinium salts have the formula

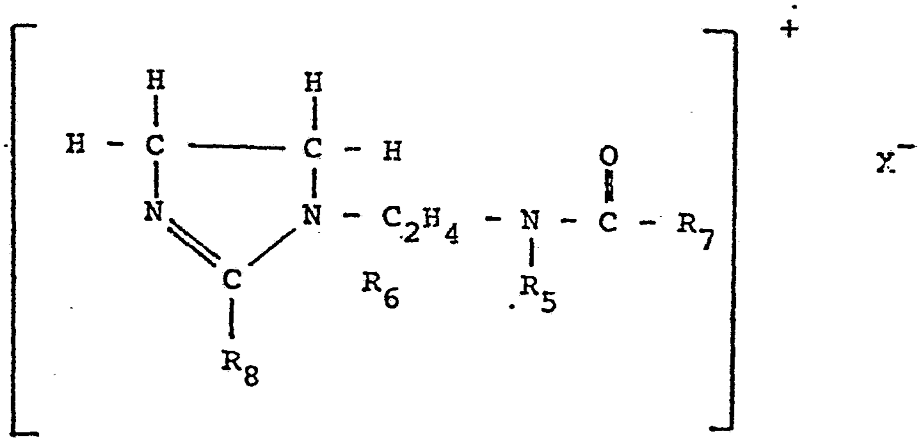

wherein $R_6$ is an alkyl group containing from 1 to 4, preferably from 1 to 2, carbon atoms; $R_5$ is an alkyl group containing from 1 to 4 carbon atoms or a hydrogen atom; $R_8$ is an alkyl group containing from 1 to 22, preferably at least 15 carbon atoms, or a hydrogen atom; $R_7$ is an alkyl group containing from 8 to 22, preferably at least 15, carbon atoms; and X is an anion, preferably chloride. Other suitable anions include those disclosed with reference to the quaternary ammonium salts described hereinbefore.

Particularly preferred are those imidazolinium salts in which both $R_7$ and $R_8$ are alkyl of from 12 to

22 carbon atoms, e.g., 1-methyl-1- [(stearoylamide)ethyl] -2-heptadecyl-4,5-dihy-droimidazolinium chloride; 1-methyl-1- [(palmitoylamide)ethyl] -2-octadecyl-4,5-dihydroimidazolinium chloride; and 1-methyl-1- [(tallowamide)-ethyl] -2-tallow-imidazolinium methyl sulfate.

The sorbitan esters are the esterified cyclic dehydration products of sorbitol. Sorbitol, itself prepared by catalytic hydrogenation of glucose, is dehydrated in well known fashion to form mixtures of cyclic 1,4- and 3,6-sorbitol anhydrides and small amounts of isosorbides. (See, for example, U.S. Patent 2,322,821 issued to Brown on June 29, 1943.) The resulting complex mixtures of cyclic anhydrides of sorbitol are collectively referred to herein as "sorbitan". It will be recognized that this "sorbitan" mixture will also contain some free uncyclized sorbitol.

Sorbitan esters useful herein are prepared by esterifying the "sorbitan" mixture with a fatty acyl group in standard fashion, e.g., by reaction with a fatty (10 to 24 carbon atoms) acid or fatty acid halide. The esterification reaction can occur at any of the available hydroxyl groups and various mono-, di-, etc., esters can be prepared. In fact, complex mixtures of mono-, di-, tri-, and tetra-esters almost always result from such reactions; the stoichiometric ratios of the reactants can simply be adjusted to favor the desired reaction product.

The foregoing complex mixtures of esterified cyclic dehydration products of sorbitol (and small amounts of esterified sorbitol) are collectively referred to herein as "sorbitan esters". Sorbitan mono- and di-esters of lauric, myristic, palmitic, stearic and behenic acids are particularly useful herein. Mixed sorbitan esters, e.g. mixtures of the foregoing esters, and mixtures prepared by esterifying

sorbitan with fatty acid mixtures such as mixed tallow and hydrogenated palm oil fatty acids, are useful herein and are economically attractive. Unsaturated 10 to 18 carbon atom-containing sorbitan esters, e.g., sorbitan mono-oleate, usually are present in such mixtures. It is to be recognized that all sorbitan esters, and mixtures thereof, which are essentially water-insoluble and which have fatty hydrocarbyl "tails", are agents in the context of the present invention.

The preferred alkyl sorbitan ester agents herein comprise sorbitan monomyristate, sorbitan monopalmitate, sorbitan monostearate, sorbitan monobehenate, sorbitan dilaurate, sorbitan dimyristate, sorbitan dipalmitate, sorbitan distearate, sorbitan dibehenate, and mixtures thereof, the mixed coconutalkyl sorbitan mono- and di-esters and the mixed tallowalkyl sorbitan mono- and di-esters. The tri- and tetra-esters of sorbitan with lauric, myristic, palmitic, stearic and behenic acids, and mixtures thereof, are also useful herein.

Another useful type of treatment agent is the substantially water-insoluble group of compounds chemically classified as fatty alcohols. Mono-ols, di-ols, and poly-ols having the requisite melting points and water insolubility properties set forth above are useful herein.

Preferred alcohols useful herein are the higher melting members of the fatty alcohol class. Although once limited to alcohols obtained from natural fats and oils, the term "fatty alcohols" has come to mean all alcohols which correspond to the alcohols obtainable from fats and oils, and all such alcohols can be made by synthetic processes. Fatty alcohols prepared by the mild oxidation of petroleum products are useful

herein. Preferred fatty alcohols are saturated and have from 14 to 18 carbon atoms.

Examples of satisfactory alcohols are 1-tricosanol, 1-tetradecanol, 1-hexadecanol, 1-heptadecanol, 1-octadecanol, 1-eicosanol, 15-methyl hexadecanol, 1-heneicosanol, 2-octadecanol, 2-eicosanol, 1,1-diphenyl hexadecanol, 4-methylbenzyl alcohol, 1,12-octadecanediol, and 1,10-decanediol.

Another type of hair conditioning agent useful in the present invention encompasses various esters of polyhydric alcohols. Polyhydric alcohols (e.g. glycerol, pentaerythritol and ethylene glycol) are reacted with a fatty acid containing 8 to 20 carbon atoms in well-known fashion to produce the polyhhydric alcohol esters. Such compounds can be fully esterified or can have one or more free hydroxyl groups provided they have a melting point as mentioned above and are substantially water-insoluble.

Esters of glycerol useful herein include the mono-, di-and tri-glycerides. The fatty acid groups on the glycerides contain from 8 to 20 carbon atoms. In particular, di-glycerides containing two 8 to 20 carbon atom, preferably 10 to 18 carbon atom, alkyl groups in the molecule are useful hair conditioning agents.

Non-limiting examples of polyhydric alcohol esters useful herein include: glycerol-1,2-dilaurate; glycerol-1,3-dilaurate; glycerol-1,2-dimyristate; glycerol-1,3-dipalmitate; glycerol-1, 2-distearate; glycerol-1,3-distearate; glycerol-1,2,3-trimyristate; butane tetra-ol-1,2,3-tristearate; ethylene glycol monostearate; and ethylene glycol distearate. Mixed glycerides available from mixed tallowalkyl fatty acids, i.e., 1,2-ditallowalkyl glycerol, 1,3-ditallowalkyl glycerol, and 1,2,3-tritallowalkyl glycerol, are

economically attractive for use herein. The foregoing esters are preferred for use herein due to their ready availability from natural fats and oils.

Fatty acids salts, especially the salts of the fatty acids having from 8 to 20 carbon atoms are used herein. Such salts are prepared by neutralizing the free fatty acids with a metallo base, e.g. $Mg(OH)_2$ or $Ca(OH)_2$, in well known fashion.

Examples of suitable fatty acid salts include calcium dodecanate, calcium tetradecanate, aluminum hexadecanate, magnesium hexadecante, calcium eicosate, calcium 5-methyloctadecanate, magnesium 6-methyloctadecanate and calcium 2-hexadecanate.

Long chain aliphatic ethers with at least one hydrocarbon moiety containing 10 to 22 carbon atoms, including mono- and poly-ether alcohols having at least one free -OH group, also are treatment agents. The ether-alcohols are prepared by the classic Williamson ether synthesis. Ethers useful herein include glycerol-1,2-dilauryl ether; glycerol-1,3-distearyl ether; butane tetra-ol-1,2,3-trioctanyl ether; ethylene glycol monolauryl ether; and propylene glycol monolauryl ether.

Mixtures of treatment agents as well as single treatment agents are within the scope of this invention and can be substituted for single agents.

Most preferred of the treatment agents is ditallow dimethylammonium chloride, hereinafter referred to as DTDMAC.

Optionally, microcrystalline waxes can be added to the treatment agent to enhance its activity. These additives improve the dry combing characteristics of a subject's hair and reduce resoiling. Examples of suitable microcrystalline waxes include common household

parafin wax and Indrawax 2025 as manufactured by the Quaker State Oil Co. of Farmers Valley, Pennsylvania.

Treatment agent is releasably applied to the first surface (or face) of the substrate. The first surface of the substrate is that surface portion of the substrate which is intended to contact a subject's hair. When both surfaces of the substrate are planar, the particular surface to which the treatment agent is applied is immaterial so far as the functioning of this invention is concerned. In this particular case, the first surface of the substrate is selected for convenience of manufacture. If, on the other hand, the substrate is in one of the preferred embodiments containing embossments, the first surface is that surface containing the embossments. That is to say, when the base of the embossments are in the plane of the substrate, the apices of the embossments must generally be in an imaginary plane adjacent one surface of the substrate. This imaginary plane is adjacent the first surface of the substrate.

Treatment agent can be applied to the first surface of the substrate by any convenient means. Preferably, the treatment agent is dissolved in an inert vehicle and is sprayed onto the first surface of the substrate and the vehicle is allowed to evaporate. In an alternative preferred application procedure, the treatment agent is melted and the molten treatment agent is sprayed or extruded onto the first surface of the substrate where it is allowed to cool and solidify. Application of the treatment agent can occur prior to, concurrent with, or subsequent to the union of the substrate and the backing sheet as hereinafter described. Preferably, the treatment agent is applied to the substrate subsequent to its union with the backing sheet.

The amount of treatment agent applied to the substrate depends upon the particular treatment agent used, the particular surface characteristics of the substrate in terms of geometry and release properties, and the desired functionality of the overall product. In general, the level of treatment agent will be from about 0.1 to about 10.0 grams per square meter of substrate surface. When the substrate comprises the especially preferred cylindrical embossments hereinbefore described, and when the treatment agent is DTDMAC, the level of treatment agent applied to the substrate is from about 0.4 to about 9.0 grams per square meter of substrate surface.

The Backing Sheet

The third of the three basic elements of this invention is the backing sheet. The backing sheet can be any sheet, film, paper, cloth, or other planar material which is flexible and which is compatible with the substrate and with the objects of this invention as a whole. The preceding sentence is intended to imply that a great deal of latitude exists in selecting the backing sheet. In the paragraphs which follow, particular desirable and necessary characteristics of the backing sheet are discussed.

It should be understood that the function of the backing sheet is to complement and enhance the function of the substrate and the treatment agent. Generally, the substrates of this invention are relatively flimsy and highly flexible plastic films which are, on the second surface thereof, slick, adherent to moistened fingers, sometimes clamy to the touch, and frequently difficult to hold and manipulate. The backing sheet serves to remedy these undesirable traits.

As noted supra, the substrate must be flexible. For economic reasons, it is frequently desirable that the substrate should be a relatively thin plastic film. It is a well-known phenomenon that relatively thin plastic films possess a high degree of flexibility. Frequently, this degree of flexibility is considerably greater than that required for proper operation of the invention. The backing sheet, which possesses some lesser degree of flexibility as well as some inherent body, or bulk, serves to decrease the excessive flexibility of the substrate. That is to say, the backing sheet adds body and decreases the flexibility of the substrate, thereby producing an overall product which is still flexible, as that term is defined above, but which is not too flexible for convenient use. The overall product must still be flexible enough so that it can be deformed by moderate-to-light manual pressure and into more or less conforming register with relatively small objects such as a lock of human hair.

A second function of the backing sheet is to provide a pleasant tactile characteristic to the product. As will be discussed more fully hereinafter, the subject will grasp the product of this invention with her hand contacting the backing sheet, leaving the first surface of the substrate which is coated with treatment agent free to contact her hair. The backing sheet will generally provide a more pleasing tactile sensation to her hand than will the second surface of the substrate.

Still another function of the backing sheet is to provide body to the overall product, thereby making the overall product more pleasant to use and more luxurious feeling than one which is composed of the substrate alone.

Suitable backing sheets include both woven and nonwoven fabrics well known in the art. While woven fabrics can be used as the backing sheet, they are generally too expensive for use and frequently provide more stiffness and body to the overall product than is required. Nonwoven fabrics, on the other hand, are much less expensive and can be procured in forms quite suitable for use herein.

Nonwoven fabrics suitable for use as backing sheets can be made from cellulosic fibers, either naturally occurring fibers such as cotton fibers or fibers made from regenerated cellulose such as rayon fibers. Nonwoven fabrics made from other synthetic fibers such as nylon can also be used.

Suitable backing sheets can also be made from sheets of low density cellulose, polyurethane, and polyester foams.

Especially preferred backing sheets are those which are made from tissue paper such as that sometimes called paper toweling. Tissue paper made from wood pulp fibers generally by the process described by Sanford and Sisson in U.S. Patent 3,301,746, issued January 31, 1967, are especially preferred. By properly controlling the fiber furnish used to make the tissue paper, by selecting the natural and synthetic binding agents, and by controlling the papermaking process, all as well known to those skilled in the papermaking art, one can make an eminently suitable backing sheet. Especially preferred are those tissue papers made from mixed hardwood and softwood kraft and sulfite wood pulps and which contain modest amounts of synthetic wet strength resins.

Physically, the backing sheet must be strong enough to withstand moderate usage. Materials having

a minimum dry tensile strength of about 40 grams per centimeter in all directions are generally suitable. Furthermore, materials used as backing sheets must be relatively soft and pleasing to the touch.

It has been discovered that when the backing sheet and the substrate are affixed one to another with substantially overall adhesive bonding as hereinafter described, that the backing sheet needs little inherent tensile and tear strength.

Optionally, the backing sheet can have decorative embossments, designs, and the like. Especially preferred for use herein are those tissue paper webs having a density of from about 0.03 to about 0.20 gram per cubic centimeter and a basis weight of from about 10 to about 40 grams per square meter.

The backing sheet is brought adjacent to and affixed to the second surface of the substrate by any convenient method well known to those skilled in the art. (The second surface, or face, of the substrate is, naturally, the surface opposite the first surface.) For example, an adhesive, such as a hot melt glue, is applied to the substrate and the substrate and backing sheet are brought into register and pressed together with sufficient force and for a sufficient time to allow a firm bond between the two elements to form. Alternatively, the adhesive can be applied to the backing sheet by any convenient means before it is affixed to the substrate as immediately hereinbefore described. The adhesive is preferably applied to either element or both elements as a film covering essentially the complete area of the substrate or the backing sheet. It can also be applied in any convenient continuous or discontinuous pattern of stripes, dots, hexagons, triangles, squares, rectangles or the like.

Preferably, the adhesives used in this invention are, after curing, soft and flexible. For economic reasons, of course, the amount of adhesive used should be as small as possible consistent with proper attachment of substrate and backing sheet. Hot melt adhesives are, for example, used at levels of from about 2 grams per square meter to about 10 grams per square meter.

Figure 8 is a fragmentary cross-sectional view of a product of this invention. Reference numerical 1 indicates the substrate, 9 the treatment agent, and 8 the backing sheet.

If the substrate is provided in continuous roll form, the backing sheet should be provided in continuous roll form. If the substrate is provided in the form of convenient sized sheets, the backing sheet should be provided in the form of convenient sized sheets.

The substrate and the backing sheet can be united prior to, simultaneously with, or subsequent to the application of the treatment agent to the first surface of the substrate. Preferably, the substrate and the backing sheet are united prior to the application of the treatment agent.

Final Product Preparation

It is to be anticipated that the product of this invention will be made by commercial scale equipment and that the composite comprising the substrate, treatment agent, and backing sheet will be much larger than is convenient for use by a subject. Consequently, the overall composite should be cut into units of a size convenient for use by a subject. For example, the three element composite can be cut into rectangular products of about 20 by about 24 centimeters. Alternatively, the three-element composite

can be cut into a continuous length of composite from about 20 to about 25 centimeters wide and can be provided to the subject as a small roll of product. In this latter situation, the subject will cut or tear a convenient length, such as from about 15 to about 25 centimeters, from the small roll. When it is finally in the desired configuration, the product can be placed into any convenient packages suitable for storage and dispensing by a subject at the point of use.

In the foregoing discussion, the product of this invention was considered to be a planar (i.e. flat, sheet-like) device. And that is a preferred embodiment. One can, however, without departing from the spirit of this invention, form the final product into a glove, a mitten, an open or closed cylinder, or any other shape. The product will function as envisioned so long as hair is contacted with treatment agent that is releasably associated with one face of a substrate while a backing sheet is associated with the other face, as described hereinbefore.

To use the product of this invention, the subject will manually grasp the product with her hand adjacent the backing sheet and will cause the product to be passed over and through her hair in such a fashion that the treatment agent is transferred from the substrate to her hair. Preferably, she has shampooed her hair- or at least dampened it - immediately before using the product of this invention.

## Test Methods

The various products of this invention, as well as similar or related products, are evaluated by the manikin detangling test. Generally, the results of this test are expressed as manikin combing ratios

(MCR). Briefly, MCR is the ratio, expressed as a percentage, of the time it takes for a qualified technician to detangle hair treated with the product under consideration (experimental time) to that time required by the technician to detangle hair which has been shampooed but not otherwise treated (base time).

The manikin used in this test is the Wave-Rite #2 supplied by Morris Flamingo Company of Danville, Illinois. This manikin has the general size and configuration of an adult human and is furnished with approximately 100grams of dark brown human hair cut to a length of 30.5 centimeters. Hair is distributed across the manikin generally as hair is normally distributed on an adult human.

To determine the detangling time, the hair on the manikin is treated in the appropriate fashion, excess water is manually squeezed from the hair, and the hair is blotted with a terrycloth towel for 30 seconds. Tangles in the hair are gently removed by combing by a technician using the large teeth of a common penny comb. The tangles are removed by combing the hair from tip to root end just as if the technician were combing her own hair. Combing is done gently to remove most tangles and is continued at a steady pace. After the majority of tangles are removed, the small teeth of the comb are used to remove any remaining tangles. A technician practices this procedure until she is able to reproduce the base or standard detangling time within ± 15 seconds. In general, the base detangling times fall between 250 and 400 seconds, depending upon the particular technician. Because manikins gradually lose hair after repeated combings, a manikin is discarded when the base time falls below 250 seconds.

The base, or standard, detangling time is determined on a manikin which has been washed with a standard shampoo. As used herein, the standard shampoo is one in which the active surfactant is the triethanolamine salt of lauryl alkyl sulfate sold under the tradename PRELL by The Procter & Gamble Company of Cincinnati, Ohio. A shampooing consists of two 30-second latherings with five millileters of shampoo containing about 55% (by weight) surfactant. The hair is rubbed between the hands during the lathering; it is rinsed with clear tepid water for 60 seconds after the first lathering and for 120 seconds after the second lathering. If the base time is to be determined, excess water is removed by blotting as above and the hair is combed to obtain the base detangling time. If hair conditioning products are to be evaluated, the product is applied to the hair which has been shampooed and rinsed as above. Specific instructions or operations for any particular hair conditioning product will, of course, be followed to the exclusion of the general procedure. The experimental detangling time is determined as above and the MCR is calculated. It is important for accurate results that the same trained technician determine both the experimental and the base detangling times.

In the case of a liquid hair conditioning product and in the absence of instructions to the contrary, a 20 millileter sample of product is distributed manually throughout the hair for 30 seconds, the hair is rinsed for 60 seconds, and excess water removed as above. The detangling time is determined as indicated.

When the hair conditioning products of the present invention are used, the hair is gently stroked (or contacted) with the side of the product to which the agent has been applied. If no specific treatment time is indicated, the hair conditioning product of this invention is used to contact the hair for a period of 45 seconds. The detangling time is determined as indicated.

Generally, five replications of each test are run. The MCR is determined from the average detangling times thus determined.

The amount of hair conditioning agent transferred to the hair from the product of this invention is determined by difference. That is, the difference between the amount of agent left on the product after use and the amount originally present is considered to be the amount transferred to the subject's hair. The amount initially present is either known from the manufacturing parameters or is determined for representative samples of a particular batch of product by the techniques described below.

Determination of the amount of agent remaining on a given portion of the product of this invention is accomplished by a two-phase titration method. The product to be assayed is carefully extracted with methanol and the amount of cationic hair conditioning agent in the solvent is determined by the method described generally by Reid et al in Tenside, Vol. 4, pages 292-304 (1967) and Vol 5, pages 90-96 (1968), both of which papers are incorporated herein by reference.

It is believed that the invention can be better understood by a reading of the following nonlimiting examples.

EXAMPLE I

A high slip, low density polyethylene film, 0.032 millimeter in thickness, as supplied by the Visqueen Division, Ethyl Corporation, Terre Haute, Indiana, was provided with obalte spheroid embossments as shown generally in Figure 7. In these embossments, d was approximately 0.82 millimeter and t was approximately 0.20 millimeter. The embossments were arranged generally as in Figure 6 with s and g approximately 0.21 millimeter. This particular embossment geometry provides an available surface area of approximately 1.37 square meters per square meter of product. The surface of the substrate was slightly roughened to reduce the glare. The substrate was joined to a single ply of tissue paper as supplied by The Procter & Gamble Company, Cincinnati, Ohio. This tissue paper backing sheet had a basis weight of about 27.3 grams per square meter and a density of approximately 0.08 gram per cubic centimeter. A hot melt glue sold under the tradename of National Starch 34-2811 by the National Starch and Chemical Company of Bridgewater, New Jersey was used to join the substrate and the backing sheet. A continuous layer of adhesive approximately 0.006 millimeter in thickness was used. In this particular example, the hair conditioning agent was DTDMAC which was extruded through a coating head onto the substrate at a level of 2.4 grams per square meter of product. The product was manufactured in the form of parent rolls of composite and individual units of product having the dimensions of 20.3 by 22.9 centimeters were cut from the finished parent rolls.

EXAMPLE I (continued)

When subjected to laboratory testing as hereinbefore described, the product of this example exhibited a MCR of 64%. Analytical determinations as hereinbefore described indicated that 64.5% of the treatment agent was transferred to the hair during the single 45-second application.

EXAMPLE II

The polyethylene film of Example I was provided with cylindrical embossments generally as shown in Figure 4. Base diameter $d_1$ was approximately 0.127 millimeter while height $h_1$ was approximately 0.076 millimeter. The embossments were arranged in regular ranks and files as generally indicated in Figure 5 with a spacing (s and g) between bases of approximately 0.127 millimeter. This particular embossment pattern resulted in an available surface area of 1.47 square meters per square meter of product. The substrate was joined as in Example I to a single ply tissue paper, as manufactured by The Procter & Gamble Company, having a basis weight of 18.3 grams per square meter and a density of 0.05 gram per cubic centimeter. The substrate was provided with 2.6 grams of DTDMAC per square meter of product. The product was manufactured as in Example I.

A performance evaluation of this product indicated that an individual sheet 22.9 centimeters x 20.3 centimeters exhibited a MCR of 65%. Approximately 48% of the treatment agent was transferred to the hair during the single 45-second application.

EXAMPLE III

The polyethylene film of Example I without embossments was laminated to the backing sheet of Example I as indicated in Example I. Naturally, the available surface area of substrate of this product was 1 square meter per square meter of product. The product was provided with 2.8 grams per square meter DTDMAC as in Example I.

The MCR determined as in Example II was 57%. Approximately 75% of the treatment agent was transferred from the product to the hair during a single 45-second application.

EXAMPLE IV

The film of Example I was provided with conical, apertured embossments as described in the hereinbefore incorporated U.S. Patent 3,929,135. The cones were approximately 0.76 millimeter in diameter at their bases and had a height of approximately 0.51 millimeter. The cones were arranged generally as shown in Figure 6, but the bases of the cones were immediately adjacent one another (i.e. s and g were zero.) This particular geometry resulted in an available surface area of 2 square meters per square meter of product. The embossed film was laminated to the backing sheet of Example I as in Example I and was provided with 3.22 grams DTDMAC per square meter of product.

When tested as in Example I, but with a single 90-second application time as opposed to a 45-second application, the product exhibited a MCR of 45%. Approximately 65% of the treatment agent was transferred from the product to the hair.

CLAIMS

1. A hair conditioning product characterized by:

(a) a flexible, impervious, nonabsorbent, planar substrate;
(b) a hair conditioning agent releasably associated with the first surface of said substrate; and
(c) a backing sheet affixed to the second surface of said substrate.

2. A product according to Claim 1 characterized in that said substrate is provided with embossments.

3. A product according to Claim 2 characterized in that said embossments are selected from the group consisting of cones, cylinders, and oblate spheroids.

4. A product according to Claim 2 or 3 characterized in that said embossments are randomly distributed.

5. A product according to Claim 2 or 3 characterized in that said embossments are distributed in a regular, repeating pattern.

6. A product according to any preceding Claim characterized in that said hair conditioning agent is selected from the group consisting of quaternary ammonium salts, fatty alcohols, sorbitan esters, polyhydric alcohol esters, fatty acid salts, aliphatic ethers, and mixtures thereof.

7. A product according to any preceding Claim characterized in that said backing sheet is selected from the group consisting of paper, woven fabric, non-woven fabric, cellulose foam, polyurethane foam, and polyester foam.

8. A product according to any preceding Claim characterized in that said hair conditioning agent is a quaternary ammonium salt and said backing sheet is paper.

9. A product according to any preceding Claim characterized in that said hair conditioning agent is ditallow dimethyl ammonium chloride.

10. A method of treating hair by manually contacting said hair with hair conditioning agent which is releasably associated with the first surface of the flexible, impervious, nonabsorbent, planar substrate of the hair conditioning product of any of Claims 1 to 9.

Fig. 1

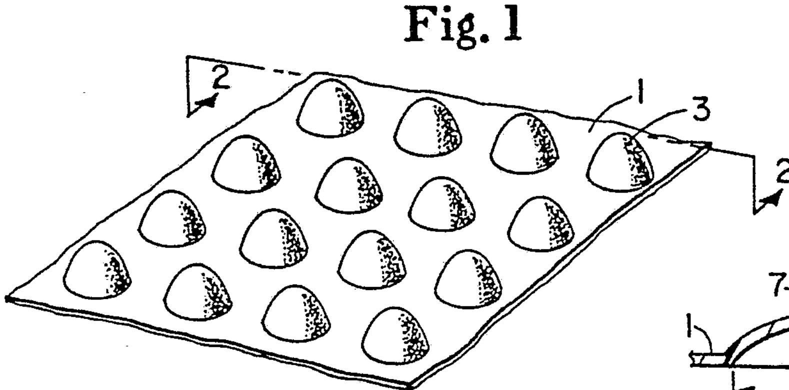


Fig. 7

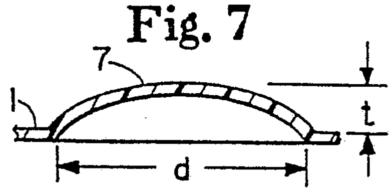


Fig. 2

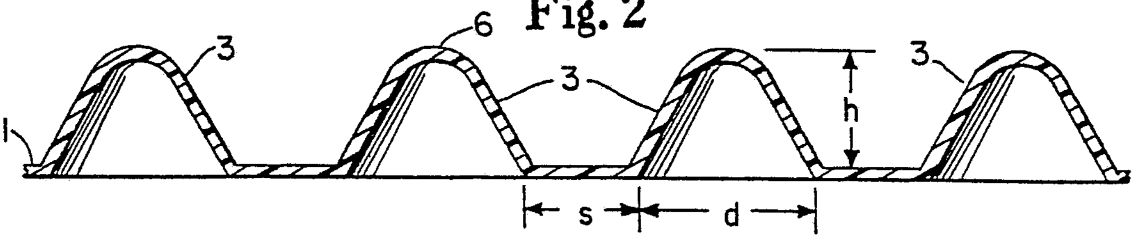


Fig. 3

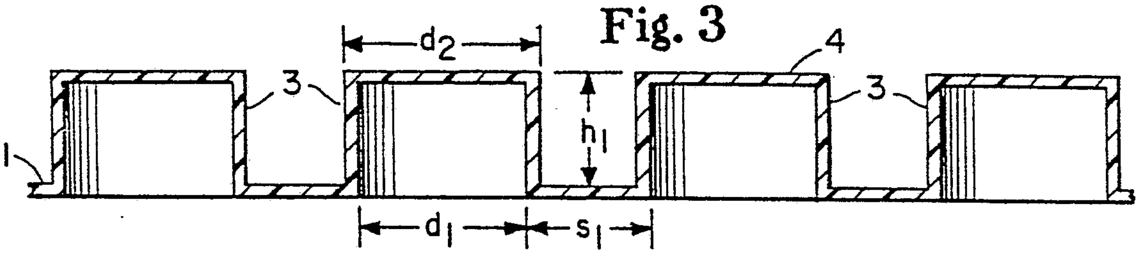


Fig. 4

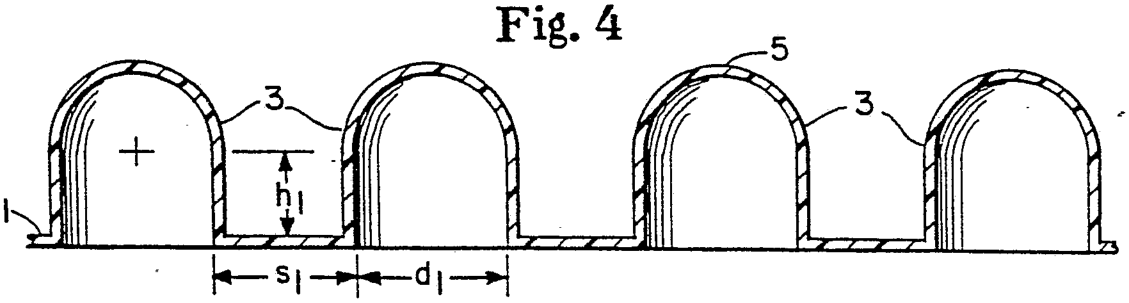


Fig. 5

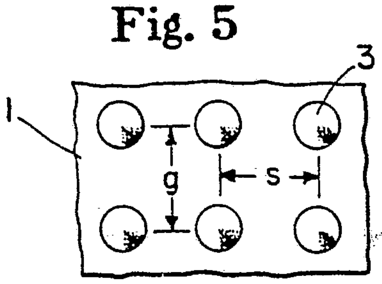


Fig. 6

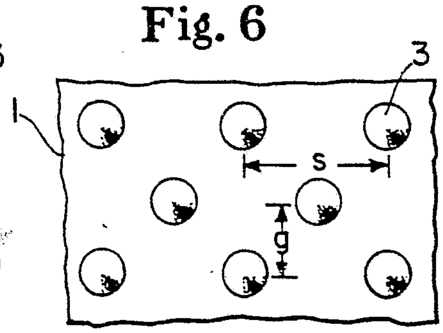


Fig. 8

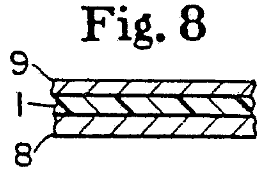

0021505

European Patent Office

# EUROPEAN SEARCH REPORT

Application number
EP 80 20 0543

| Category | Citation of document with indication, where appropriate, of relevant passages | Relevant to claim | CLASSIFICATION OF THE APPLICATION (Int. Cl.3) |
|---|---|---|---|
| | US - A - 3 992 336 (FAUCHER et al.) * Column 1, line 20 - column 2, line 62; claims * | 1,10 | A 61 K 7/06 |
| A | GB - A - 1 304 375 (L'OREAL) * Claims 1,4 * | 1 | |
| A | FR - A - 958 219 (A. BILLY) * Figure; abstract, points I, 1°,2° * | 2 | |
| A | US - A - 3 954 113 (BOHRER et al.) * Claims 1,8-10,16; column 5, lines 59-68; column 6, line 67 - column 7, line 54 * | 1,10 | |
| P | GB - A - 2 022 415 (PROCTER & GAMBLE) * Claims 1,2,9,10 * | 1,6,9,10 | |
| P | GB - A - 2 024 623 (PROCTER & GAMBLE) * Claims 1,2,9,11 * | 1,6,9,10 | |

TECHNICAL FIELDS SEARCHED (Int. Cl.3)

A 61 K 7/00
7/06
A 45 D 7/04
33/38

CATEGORY OF CITED DOCUMENTS

X: particularly relevant
A: technological background
O: non-written disclosure
P: intermediate document
T: theory or principle underlying the invention
E: conflicting application
D: document cited in the application
L: citation for other reasons

&: member of the same patent family, corresponding document

X The present search report has been drawn up for all claims

| Place of search | Date of completion of the search | Examiner |
|---|---|---|
| The Hague | 26-09-1980 | WILLEKENS |

EPO Form 1503.1 06.78